(19) Europäisches Patentamt
European Patent Office
Office européen des brevets

(11) Numéro de publication : **0 044 771**
**B1**

(12) # FASCICULE DE BREVET EUROPÉEN

(45) Date de publication du fascicule du brevet : **02.05.84**

(21) Numéro de dépôt : **81401115.1**

(22) Date de dépôt : **09.07.81**

(51) Int. Cl.³ : **B 01 J 31/24**, C 07 B 27/00// C07C49/203, C07C49/24, C07C69/738, C07C147/08, C07C175/00, C07C45/69, C07C67/347

(54) **Procédé d'addition sélective d'un composé ayant un atome de carbone activé sur un diène conjugué substitué.**

(30) Priorité : **10.07.80 FR 8015355**
**11.05.81 FR 8109322**

(43) Date de publication de la demande :
**27.01.82 Bulletin 82/04**

(45) Mention de la délivrance du brevet :
**02.05.84 Bulletin 84/18**

(84) Etats contractants désignés :
**AT BE CH DE FR GB IT LI LU NL SE**

(56) Documents cités :
**DE-A- 2 350 194**
**FR-A- 2 366 237**
**JOURNAL OF THE CHEMICAL SOCIETY, Perkin Transactions II, Physical Organic Chemistry pages 1133-1138, 1975 R. BAKER et al.: "Reactions of Amines and Active Methylene Compounds with Buta-1,3-diene and Isoprene: Catalysis by Nickel, Cobalt, Rhodium, and Iridium Complexes"**
**TETRAHEDRON LETTERS, no. 38, pages 3575-3578, septembre 1978 Pergamon Press, GB R. BAKER et al.: "Reactions of Active Methylene and Carbonyl Compounds with Myrcene Catalysed by Palladium and Nickel Complexes"**
**BULLETIN OF THE CHEMICAL SOCIETY OF JAPAN, vol. 48, no. 6, June 1975, page 1949 H. KAWAZURA et al.: "The Regioselective Addition of Alcohols to Isoprene with Rhodium Trichloride Hydrates"**

(73) Titulaire : **RHONE-POULENC SANTE**
**Les Miroirs 18 Avenue d'Alsace**
**F-92400 Courbevoie Cedex (FR)**

(72) Inventeur : **Morel, Didier**
**19, avenue Jean Mermoz**
**F-69008 Lyon (FR)**

(74) Mandataire : **Pilard, Jacques et al**
**RHONE-POULENC RECHERCHES Service Brevets Pharma 25, Quai Paul Doumer**
**F-92408 Courbevoie Cedex (FR)**

**EP 0 044 771 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé d'addition sélective d'un composé ayant un atome de carbone activé sur un diène conjugué substitué

La présente invention a pour objet un procédé d'addition sélective d'un composé ayant un atome de carbone activé sur un diène conjugué ; elle concerne plus particulièrement l'addition sélective d'un composé ayant un atome de carbone activé sur l'atome de carbone 4 d'un butadiène-1,3 portant un substituant sur l'atome de carbone 2.

On connaît dans l'art antérieur le brevet français 7 622 824 au nom de la demanderesse publié sous le numéro 2 366 237. Ce brevet décrit la réaction d'au moins une mole de diène avec un composé à hydrogène mobile. Selon ce brevet, on effectue la réaction en présence d'un catalyseur constitué, d'une part, par une phosphine soluble dans l'eau et, d'autre part, par un métal de transition (sous forme métallique ou sous forme d'un composé) et on introduit de l'eau dans le milieu réactionnel, le catalyseur passant en solution dans l'eau. Le métal de transition est choisi parmi le groupe comprenant le palladium, le nickel, le platine, le cobalt et le rhodium ; le palladium étant le métal préféré.

Il est précisé dans ce brevet que lorsque l'on fait réagir une mole de diène, par exemple le butadiène, avec une mole de composé à hydrogène mobile, on obtient principalement les composés suivants :

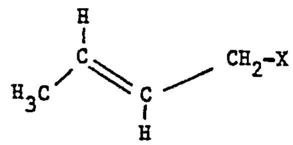 et 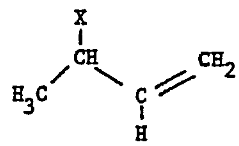

Poursuivant ses recherches, la demanderesse a axé ses travaux sur l'obtention des composés résultant de l'addition sur le carbone 4 d'un butadiène-1,3 portant un substituant hydrocarboné R sur le carbone 2, c'est-à-dire le butadiène de formule :

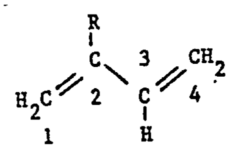

(I)

d'un composé ayant un atome de carbone activé de formule :

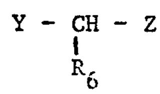

(II)

dans laquelle $R_6$ représente un atome d'hydrogène ou un radical hydrocarboné et l'un des symboles Y et Z est un groupe électro-attracteur et l'autre représente un groupement électro-attracteur ou électro-donneur et lorsque l'un des symboles Y ou Z représente un radical acyle, celui-ci peut former avec $R_6$ un cycle saturé à 5 ou 6 chaînons.

Ces composés ont en effet un réel intérêt industriel et économique dans la mesure où ils sont utilisés en tant que précurseurs d'intermédiaires pour la synthèse de vitamines et de parfums.

La demanderesse a constaté que la mise en œuvre du procédé décrit dans le brevet de l'art antérieur précité ne peut être transposé directement pour la préparation de ces composés d'addition.

En effet, la demanderesse a constaté que dans le cas de la réaction des composés I et II, le diène n'étant pas symétrique, il se forme, lorsque l'on utilise comme métal de transition l'un des métaux suivants : palladium, nickel, platine et cobalt, quatre composés correspondant à l'addition sur les 4 carbones du butadiène ; on obtient donc un mélange des composés suivants :

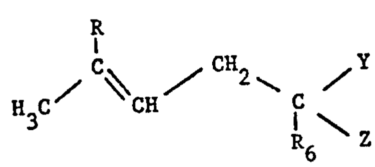

(A)

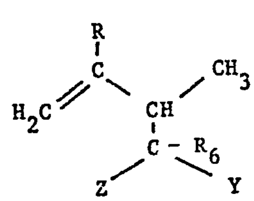

(B)

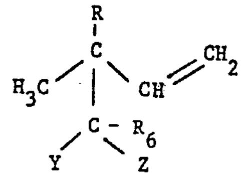

(C)

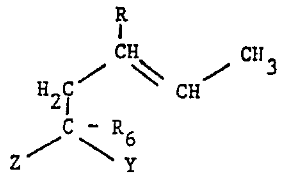

(D)

D'autre part, il ressort des expériences conduites par la demanderesse que plus le substituant R est important, plus la quantité de produits A, B, C et D qui se forme est faible, quand on utilise les métaux de transition palladium, nickel, platine et cobalt.

C'est ainsi que lorsque le composé de formule (I) est le myrcène, il se forme une quantité très faible de produit, ce qui rend le procédé totalement impropre à une exploitation industrielle.

Lorsque la production est plus importante, la séparation des produits de la réaction est difficile, longue et coûteuse.

La demanderesse a constaté que lorsque l'on veut faire réagir le composé I et le composé II, seule l'utilisation du rhodium comme métal de transition permet d'obtenir une addition totalement sélective sur le carbone 4, cela quelle que soit la taille du substituant hydrocarboné R et une activité très nettement améliorée par rapport aux autres métaux mentionnés dans le brevet précité de l'art antérieur.

L'invention permet donc la préparation sélective d'un composé dans des conditions techniques et économiques très avantageuses.

L'invention a donc pour objet un procédé d'addition d'un composé de formule (II) ayant un atome de carbone activé sur un butadiène-1,3 de formule (I) portant un substituant hydrocarboné sur l'atome de carbone 2, procédé du type selon lequel on fait réagir le composé ayant un atome de carbone activé et le butadiène-1,3 portant un substituant hydrocarboné dans l'eau ou dans un milieu hydroalcoolique contenant au maximum 50 % d'un alcool aliphatique contenant 1 à 3 atomes de carbone en présence d'un catalyseur constitué, d'une part, par au moins une phosphine soluble dans l'eau et, d'autre part, par au moins un composé d'un métal de transition, le catalyseur étant en solution dans l'eau, procédé caractérisé en ce que, dans le but d'obtenir une addition totalement sélective du composé ayant unatome de carbone activé sur le carbone 4 du butadiène-1,3 portant un substituant hydrocarboné sur le carbone 2, le composé du métal de transition mis en œuvre est un composé du rhodium.

La demanderesse a constaté que la mise en œuvre du procédé ci-dessus conduisait d'une part à l'obtention du composé A ci-dessus mais également, et ceci est en soi très surprenant, à l'obtention des composés (A') nouveaux suivants :

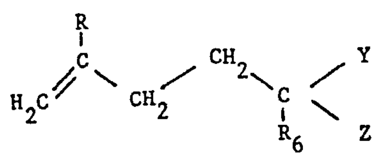

(A')

L'invention a donc également pour objet les composés (A') où R, Y et Z ont la signification précédente.

Les composés A et A' peuvent être utilisés seuls ou en mélange pour les applications susmentionnées.

Selon l'invention, dans les formules I, A et A', R représente un radical choisi parmi le groupe comprenant les radicaux alkyle ayant de 1 à 20 atomes de carbone et les radicaux phényle et naphtyle éventuellement substitués par des radicaux alkyles ayant de 1 à 20 atomes de carbone et $R_6$ représente un atome d'hydrogène ou un radical hydrocarboné défini comme R ci-dessus ; $R_6$ et R pouvant être identiques ou différents.

On peut citer comme exemples de radicaux alkyle, les radicaux $C_nH_{2n+1}$, $C_nH_{2n-1}$, $C_nH_{2n-3}$ et $C_nH_{n-1}$ où n est compris entre 1 et 20 comme par exemple : $-CH_3$ (isoprène), $-C_2H_5$, $-C_4H_9$, $-C_8H_{17}$, $-C_{20}H_{41}$ ; $-C_4H_7$, $-C_6H_{11}$ (myrcène), $-C_8H_{15}$, $C_{10}H_{19}$ ; $-C_8H_{13}$, $-C_{11}H_{19}$ (β-farnésène) ; $-C_2H$, $-C_4H_3$, $-C_8H_7$, le nom commun des composés de formule I les plus importants étant indiqués entre parenthèses.

On peut citer comme exemples de radicaux phényle et naphtyle éventuellement substitués, les radicaux tolyle, xylyle, méthyl-8 naphtyle-1 et diméthyl-5,8 naphtyle-2.

Selon l'invention, plus particulièrement, dans les formules II, A' et A, un des symboles Y et Z est

choisi parmi le groupe électro-attacteur comprenant les radicaux de formule CHO, $COR_1$, $CO_2R_2$, $SO_2R_3$, $CONR_4R_5$, CN, $NO_2$ et l'autre est choisi parmi le groupe électro-attracteur défini précédemment ou le groupe électro-donneur comprenant les radicaux de formules $NHCOR_7$, $OR_8$, OH, $OCOR_9$, $SR_{10}$, SH, les atomes d'halogène, dans lesquels $R_1$, $R_2$, $R_3$, $R_4$, $R_5$, $R_7$, $R_8$, $R_9$ et $R_{10}$ représentent un radical hydrocarboné contenant 1 à 12 atomes de carbone. Lorsque Y et Z représentent l'un un groupement électro-attracteur et l'autre un groupement électro-donneur, ils doivent être choisis de telle manière que l'effet du groupement électro-attracteur soit prépondérant.

On peut citer comme exemples de composés II les composés suivants : la pentanedione-2,4 $CH_3COCH_2COCH_3$ ; la butanal-1 one-3 $CH_3COCH_2CHO$ ; l'acétyl acétate d'éthyle $CH_3COCH_2COOC_2H_5$ ; l'acétyl acétate de méthyle $CH_3COCH_2COOCH_3$, la phénylsulfonyl acétone $C_6H_5SO_2CH_2COCH_3$, la phénylsulfonylacétate d'éthyle $C_6H_5SO_2CH_2CO_2C_2H_5$, l'éthylsulfonylacétone $C_2H_5SO_2CH_2COCH_3$, $CH_3COCH_2CON(CH_3)_2$, $CH_3COCH_2CON(CH_3)C_2H_5$, le cyanoacétate d'éthyle $NCCH_2CO_2C_2H_5$, la cyanoacétone $NCCH_2COCH_3$, le nitroacétate d'éthyle $NO_2CH_2CO_2C_2H_5$ la nitroacétone $NO_2CH_2COCH_3$, le malonate de diéthyle $CH_2(CO_2C_2H_5)_2$, l'hydroxyacétone $HOCH_2COCH_3$, l'acétoïne $HOCH(CH_3)COCH_3$, l'éthoxycarbonyl-2 cyclohexanone.

Les phosphines solubles dans l'eau utilisables dans le cadre de la présente invention sont celles décrites dans le brevet français 7 622 824 précité.

Plus particulièrement, on préfère utiliser au moins une phosphine de formule :

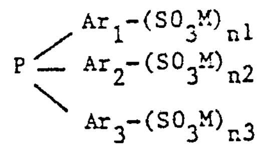

(III)

dans laquelle :

— $Ar_1$, $Ar_2$ et $Ar_3$ identiques ou différents représentent chacun un radical choisi parmi le groupe comprenant les radicaux phénylène et les radicaux naphtylène, ces radicaux étant éventuellement substitués.

— M est un reste cationique d'origine minérale ou organique choisi de manière que la phosphine de formule I soit soluble dans l'eau.

— $n_1$, $n_2$ et $n_3$ identiques ou différents sont des nombres entiers supérieurs ou égaux à 0 et inférieurs ou égaux à 3, l'un au moins étant supérieur ou égal à 1.

Les radicaux phénylène et naphtylène peuvent être substitués par tous radicaux ne contrariant pas la solubilité de la phosphine (III) dans l'eau. Parmi ceux-ci, on peut citer, à titre d'exemples, les radicaux alkyle ayant de 1 à 6 atomes de carbone, les radicaux alkoxy ayant de 1 à 6 atomes de carbone, les atomes d'halogène, les radicaux —OH, —CN, —$NO_2$, —N—(alkyl)$_2$, carboxylates.

Le procédé selon l'invention est de préférence mis en œuvre en utilisant au moins une phosphine de formule (III) dans laquelle $Ar_1$, $Ar_2$ et $Ar_3$ identiques ou différents représentent chacun un radical choisi parmi le groupe comprenant le radical phénylène et les radicaux phénylène substitués.

Encore plus préférentiellement, on utilise une phosphine dans laquelle au moins un des groupements $SO_3M$ est en position méta sur le noyau benzénique.

De préférence, M est choisi parmi le groupe comprenant les cations dérivés des métaux Na, K, Ca, Ba, les ions $NH_4^+$ et ammonium quaternaire comme les ions tétraméthylammonium, tétrapropylammonium et tétrabutylammonium.

$n_1$, $n_2$ et $n_3$ sont de préférence égaux à 0 ou 1, $n_1 + n_2 + n_3$ étant compris entre 1 et 3 ($1 \leq n_1 + n_2 + n_3 \leq 3$).

Les composés de formule III plus particulièrement préférés sont les phosphines de formules suivantes :

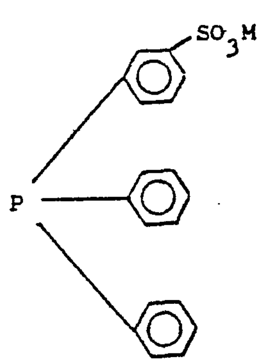

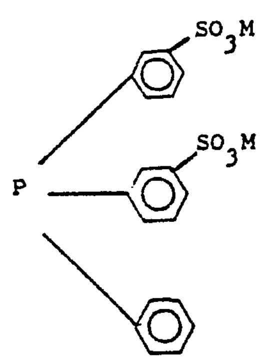

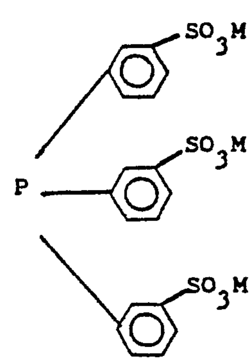

où M a la signification précédente.

On peut citer comme autres exemples de phosphine (III) pouvant être utilisées dans le procédé selon

l'invention les composés suivants : les sels alcalins ou alcalino-terreux, les sels d'ammonium, les sels d'ammonium quaternaires des (p-sulfophényl) diphénylphosphine ; (m-sulfo p-méthylphényl) di(p-méthylphényl)phosphine ; (m-sulfo p-méthoxyphényl) di(p-méthoxyphényl)phosphine ; (m-sulfo p-chlorophényl) di(p-chlorophényl)phosphine ; di(p-sulfophényl)phénylphosphine ; di(m-sulfo p-méthylphényl) (p-méthylphényl)phosphine ; di(m-sulfo p-méthoxyméthyl) (p-méthoxyphényl)phosphine ; di(m-sulfo p-chlorophényl) (p-chlorophényl)phosphine ; tri(p-sulfophényl)phosphine ; tri(m-sulfo p-méthylphényl) phosphine ; tri(m-sulfo p-méthoxyphényl)phosphine ; tri(m-sulfo p-méthoxyphényl)phosphine ; tri(m-sulfo p-chlorophényl)phosphine ; (o-sulfo p-méthylphényl) (m-sulfo p-méthyl) (m, m'-disulfo p-méthyl) phosphine ; (m-sulfophényl) (m-sulfo p-chlorophényl) (m,m'-disulfo p-chlorophényl)phosphine.

Le composé du rhodium utilisé doit être soluble dans l'eau ou capable de passer en solution dans l'eau dans les conditions de la réaction, par réaction de coordination avec les phosphines hydrosolubles. Le reste lié au métal n'est pas critique dès lors qu'il satisfait à ces conditions.

Selon un mode de réalisation préférentiel du procédé le dérivé du rhodium est choisi parmi le groupe comprenant les sels inorganiques, organiques et les complexes du rhodium comme par exemple $RhCl_3$, $RhBr_3$, $Rh_2O$, $Rh_2O_3$, $Rh(NO_3)_3$, $Rh(CH_3COO)_3$, $Rh(CH_3COCHCOCH_3)_3$, $[RhCl(\text{cyclooctadiène-1,5})]_2$, $[RhCl(CO)_2]_2$, $RhCl_3(C_2H_5NH_2)_3$.

On préfère tout particulièrement utiliser $RhCl_3$ et $[RhCl(\text{cyclooctadiène-1,5})]_2$.

On utilise une quantité de rhodium ou de composé du rhodium telle que le nombre d'atomes-gramme de rhodium élémentaire par litre de solution réactionnelle soit compris entre $10^{-4}$ et 1. De préférence, il est compris entre 0,001 et 0,5.

Pour une bonne mise en œuvre du procédé, la quantité de phosphine est choisie de telle sorte que le nombre d'atomes gramme de phosphore trivalent rapporté à un atome-gramme de rhodium soit compris entre 0,1 et 200. De préférence, ce nombre est compris entre 3 et 100.

Bien que cela ne soit pas impératif, on peut ajouter au milieu réactionnel, un réducteur de rhodium. Ce réducteur qui peut être organique ou minéral est mis en œuvre dans le but d'activer dans certains cas la formation d'espèce catalytique active.

On peut citer comme exemples de réducteurs utilisables le borohydrure de sodium, la poudre de zinc, le borohydrure de potassium, la poudre de zinc, le borohydrure de potassium, le magnésium et l'hydrazine. Ce réducteur est ajouté en quantité telle que le nombre d'équivalents oxydo-réduction est de préférence compris entre 1 et 10.

Selon un mode de mise en œuvre particulier mais non obligatoire de l'invention, on ajoute au milieu réactionnel une base dans le but d'améliorer la réactivité. On peut citer comme exemples de bases convenant particulièrement bien, les hydroxydes, carbonates et bicarbonates des métaux alcalins et alcalino-terreux et les amines tertiaires aliphatiques ou aromatiques. On utilise de préférence entre 0,005 et 5 moles de base/litre de solution aqueuse.

La température à laquelle est conduite la réaction peut varier dans de larges limites. Préférentiellement, on opère à des températures modérées inférieures à 200 °C. Plus particulièrement, la température est comprise entre 50 °C et 125 °C.

Le rapport molaire du composé II au composé I n'est pas critique. On préfère opérer avec un léger excès molaire de composé II (composé ayant un atome de carbone activé).

La quantité d'eau minimum nécessaire est celle suffisante pour dissoudre le catalyseur en totalité et au moins une partie des réactifs I et II, la réaction ayant lieu en phase aqueuse, les produits de la réaction étant en phase organique non miscible à l'eau.

Afin d'augmenter la cinétique de la réaction et de faciliter le recyclage du catalyseur, il est possible d'opérer en présence d'un co-solvant. Plus particulièrement une partie de l'eau nécessaire à la mise en œuvre de la réaction est remplacée par une quantité équivalente d'un alcool aliphatique contenant 1 à 3 atomes de carbone, tel que le méthanol, l'éthanol ou l'isopropanol. La quantité d'eau qui peut être remplacée est au maximum égale à la moitié de la quantité d'eau nécessaire à la mise en œuvre de la réaction sans co-solvant.

Une manière pratique de mettre en œuvre le procédé de l'invention consiste à charger dans un réacteur convenable après l'avoir purgé à l'aide d'un gaz inerte (azote, argon) soit la solution aqueuse ou hydroalcoolique catalytique préalablement formée, soit les divers composants : phosphine, eau, éventuellement l'alcool tel que le méthanol, l'éthanol ou l'isopropanol, composé du rhodium avec éventuellement le réducteur et la base. On porte le réacteur à la température de réaction avant ou après l'introduction du composé ayant un atome de carbone activé qui lui-même peut être introduit avant, après ou simultanément au butadiène substitué.

Après arrêt de la réaction, on refroidit à la température ambiante. Le contenu du réacteur est soutiré et on isole ensuite le produit de la réaction qui est en phase organique en séparant cette dernière de la phase aqueuse contenant le catalyseur par décantation et éventuellement par une extraction à l'aide d'un solvant convenable.

La solution aqueuse ou hydroalcoolique résiduelle peut être recyclée dans le réacteur pour catalyser une nouvelle réaction. En particulier, en solution hydro-méthanolique le catalyseur peut être recyclé plus de 25 fois sans perte d'activité. La solution aqueuse ou hydroalcoolique peut aussi rester dans le réacteur, les produits organiques étant alors soutirés après décantation.

Le procédé selon l'invention permet d'obtenir des sélectivités voisines de 100 % en composés A et A'.

Ces deux composés peuvent être séparés par tous moyens connus de l'homme de l'art, comme par exemple par distillation.

Les produits de formule générale A' obtenus selon le procédé de la présente invention sont des produits nouveaux qui peuvent, en opérant en particulier dans les conditions du brevet français 7 007 199 publié sous le numéro 2 031 335, être isomérisés en produit de formule générale A. Généralement cette isomérisation peut s'effectuer en opérant à une température voisine de 25 °C en présence d'un catalyseur tel que le palladium sur charbon.

Les exemples suivants feront apparaître d'autres caractéristiques et avantages de l'invention. Ils ne sauraient être considérés comme limitant de façon quelconque l'invention.

Exemples 1 à 11

Addition sur l'isoprène de la pentanedione 2-4 pour obtenir

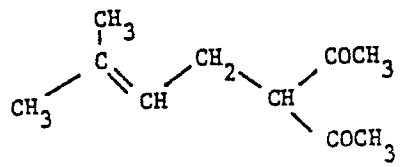

(a)

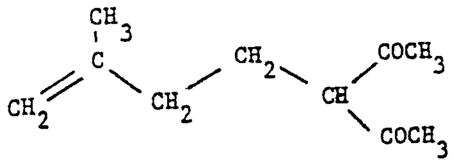

(a')

Exemple 1

Dans un autoclave en acier inoxydable préalablement purgé à l'argon, on introduit 68,4 mg de $[RhCl(cyclooctadiène\text{-}1,5)]_2$ soit 0,27 milliatome-gramme de rhodium, 0,30 g de :

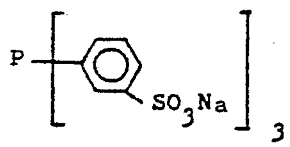

(que l'on appelera dans les exemples suivants TPPTS Na) soit 0,44 milliatome-gramme de $p^{3+}$, 0,15 g (1,4 millimole) de $Na_2Co_3$ et 10 $cm^3$ d'$H_2O$. On introduit ensuite 32 millimoles (2,18 g) d'isoprène et 38 millimoles (3,8 g) de pentanedione-2,4.

On chauffe à 80 °C sous agitation pendant 6,5 heures. Après refroidissement, on transvase le contenu de l'autoclave dans une ampoule à décanter où une phase aqueuse et une phase organique se séparent. La phase aqueuse recueillie, colorée en rouge foncé, contient le catalyseur et la phase organique supérieure exempte de catalyseur incolore contient après analyse par RMN, spectrographie de masse, IR et dosage par chromatographie en phase gazeuse :

2,49 g de a'
2,26 g de a

ce qui correspond à un taux de transformation de l'isoprène de 89,4 % et à un taux de transformation de la pentanedione-2,4 de 74,8 %. La sélectivité en a + a' est de 99 % par rapport à chaque réactif.

Exemple 2

En opérant comme dans l'exemple 1 mais en n'utilisant que 35,2 mg de $[RhCl(cyclooctadiène\text{-}1,5)]_2$ soit 0,14 milliatomegramme de rhodium, on obtient en fin de réaction :

2,57 g de a' et
2,25 g de a

ce qui correspond à un taux de transformation de l'isoprène de 91,5 %, de la pentanedione-2,4 de 76,3 %. La sélectivité en a + a' est 99 % par rapport à chaque réactif.

Essai comparatif

On opère comme ci-dessus mais en utilisant à la place de [RhCl(cyclooctadiène-1,5]$_2$ du $PdCl_2$ (25 mg soit 0,14 milliatome-gramme de de Palladium).

On obtient :

— 0 g de a'
— 1,83 g de a
— 0,142 g de composé d'addition sur le carbone 1
— 0,163 g de composé d'addition sur le carbone 2
— 0,035 g de composé d'addition sur le carbone 3 et
— 0,173 g de produits lourds

soit un taux de transformation de l'isoprène de 41,5 %, de la pentanedione 33,9 %, la sélectivité en a étant de 79 %.

Exemple 3

On opère comme dans l'exemple 1 mais en utilisant :

— 37,2 mg de [RhCl(cyclooctadiène-1,5)]$_2$ soit 0,15 milliatome-gramme de rhodium
— 60,3 millimoles (4,1 g) d'isoprène et
— 76,9 millimoles (7,7 g) de pentanedione-2,4

On obtient :

— 5,12 g de a'
— 4 g de a

soit un taux de transformation de l'isoprène de 90,7 %, de la pentanedione-2,4 de 70,9 %, la sélectivité en a + a' étant de 98 %.

Exemple 4

On opère comme dans l'exemple 1 mais en utilisant 49,6 mg de $RhCl_3$ hydraté soit 0,14 milliatome-gramme de rhodium.

On obtient :

— 2,4 g de a' et
— 1,85 g de a

soit un taux de transformation de l'isoprène de 80,8 %, de la pentanedione-2,4 de 65,4 %, la sélectivité en a + a' étant de 99 %.

Exemple 5

On opère comme dans l'exemple 1 mais en utilisant 10,8 mg de [RhCl(cyclooctadiène-1,5)]$_2$ soit 0,044 milliatome-gramme de Rh et 99,6 mg de TPPTS Na soit 1,47 milliatome-gramme de $P^{3+}$.

On obtient :

— 2,26 g de a'
— 1,78 g de a

soit un taux de transformation de l'isoprène de 76,7 %, de la pentanedione-2,4 de 60,7 %, la sélectivité en a + a' étant de 99 %.

Exemple 6

On opère comme dans l'exemple 5 mais en opérant pendant 3 heures au lieu de 6,5 heures.

On obtient :

— 0,5 g de a'
— 0,45 g de a

soit un taux de transformation de l'isoprène de 21 %, de la pentanedione-2,4 de 16 %, la sélectivité en a + a' étant de 98,3 %.

Exemple 7

On opère comme dans l'exemple 6 mais en augmentant la température à 100 °C.
On obtient :

— 2,36 g de a'
— 2,14 g de a

soit un taux de transformation de l'isoprène de 85,9, de la pentanedione-2,4 de 70,1 %, la sélectivité en a + a' étant de 98 %.

Exemple 8

On opère comme dans l'exemple 5 mais en utilisant 330 mg de TPPTS Na soit 0,50 milliatome-gramme de $P^{3+}$.
On obtient :

— 2,12 g de a'
— 1,82 g de a,

soit un taux de transformation de l'isoprène de 76,4 %, de la pentanedione-2,4 de 61,1 %, la sélectivité en a + a' étant de 99 %.

Exemple 9

On opère comme dans l'exemple 7 mais sans utiliser de $Na_2Co_3$.
On obtient :

— 0,39 g de a'
— 0,37 g de a

soit un taux de transformation de l'isoprène de 14,3 % de la pentanedione-2,4 de 11,6 %, la sélectivité en a + a' étant de 100 %.

Exemple 10

On opère comme dans l'exemple 1 mais en utilisant 10,8 mg de $[RhCl(cyclooctadiène-1,5)]_2$ soit 0,044 milliatome-gramme de Rh, 100 mg de TPPTS Na soit 0,148 milliatome-gramme de $P^{3+}$, 20 $cm^2$ d'$H_2O$, 64,1 millimoles (4,36 g) d'isoprène et 76 millimoles (7,60 g) de pentanedione-2,4.
On obtient :

— 1,78 g de a'
— 1,46 g de a

soit un taux de transformation d'isoprène de 31,5 % et de la pentanedione de 25,1 %, avec une sélectivité en a + a' de 100 %.

Exemple 11

On opère comme dans l'exemple 10 mais avec 10 $cm^3$ d'$H_2O$ et à 120 °C pendant 2 heures.
On obtient :

— 3,46 g de a'
— 3,11 g de a

soit un taux de transformation de l'isoprène de 65,5 % et de la pentanedione-2,4 de 50,6 %, avec une sélectivité en a + a' de 98,5 %.

Exemples 12 et 13

Addition sur l'isoprène de l'acétylacétate d'éthyle pour obtenir :

(Voir Schéma, page 9)

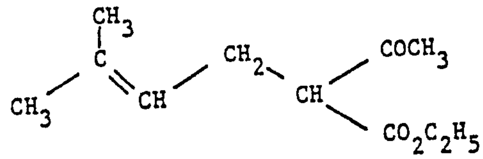

(a₁)

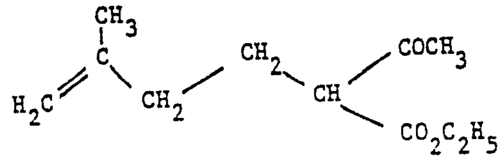

(a'₁)

Exemple 12

Dans un autoclave en acier inoxydable préalablement purgé à l'argon, on introduit 33,7 mg de $[RhCl(cyclooctadiène\text{-}1,5)]_2$ (0,14 milliatome-gramme de rhodium), 0,3 g de TPPTS Na (0,44 milliatome-gramme de $P^{3+}$), 76 mg de $Na_2CO_3$ (0,72 millimole) et 30 cm$^3$ d'$H_2O$. On introduit ensuite 102,3 millimoles d'isoprène (6,9 g) et 125 millimoles d'acétylacétate d'éthyle (16,2 g). On chauffe sous agitation à 100 °C pendant 3 heures.

On obtient :

— 6,21 g de $a_1$
— 11,9 g de $a'_1$

soit un taux de transformation de l'isoprène de 90,4 %, de l'acétylacétate d'éthyle de 73,7 %, la sélectivité en a' + $a'_1$ étant de 99 %.

Exemple 13

On opère comme dans l'exemple 12 en utilisant 132 mg de $[RhCl(cyclooctadiène\text{-}1,5)]_2$ (0,53 milliatome-gramme), 1,21 g de TPPTS Na (1,79 milliatome-gramme de $P^{3+}$), 0,75 g de $Na_2Co_3$ (7,1 millimoles), 120 cm$^3$ d'$H_2O$, 0,77 mole d'isoprène (52,4 g) et 0,88 mole d'acétylacétate d'éthyle (114 g).

On obtient :

— 38,6 g de $a_1$ et
— 81,2 g de $a'_1$

soit un taux de transformation de l'isoprène de 79,8 %, de l'acétylacétate d'éthyle de 69 %, la sélectivité en a + a' étant de 99 %.

Exemple 14

Addition sur l'isoprène de la phénylsulfonyl acétone pour obtenir :

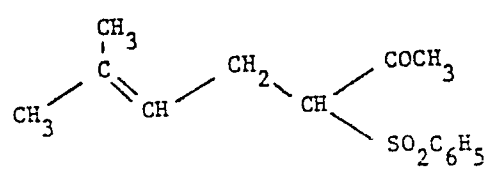

(a₂)

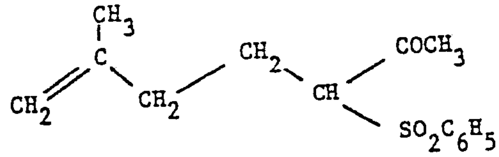

(a'₂)

Dans un autoclave en acier inoxydable préalablement purgé à l'argon, on introduit 36,6 mg de $[RhCl(cyclooctadiène\text{-}1,5)]_2$ (0,15 milliatome-gramme de rhodium), 0,32 g de TPPTS Na (0,47 milliatome-gramme de $P^{3+}$), 0,15 g de $Na_2CO_3$ (0,4 millimole) et 10 cm$^3$ d'$H_2O$.

On introduit ensuite 33,2 millimoles d'isoprène (2,25 g) et 40 millimoles de phénylsulfonylacétone (7,9 g).

On chauffe à 100 °C pendant 3 h sous agitation.

On obtient :

— 3,89 g de $a_2$
— 4,76 g de $a'_2$

soit un taux de transformation de l'isoprène de 97,9 %, de la phénylsulfonylacétone de 81,3 %, la sélectivité en $a_2 + a'_2$ étant de 99 %.

Exemples 15 à 17

Addition sur le myrcène de la pentanedione-2,4 pour obtenir :

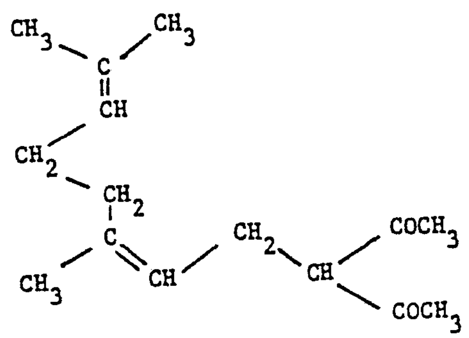

($a_3$)

et

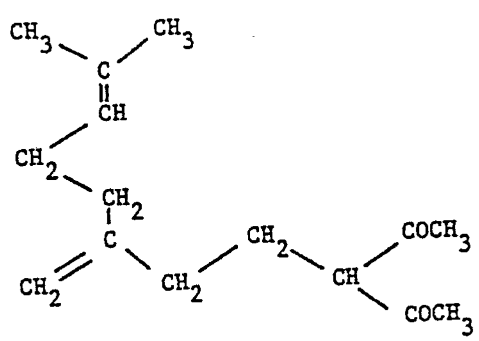

($a'_3$)

Exemple 15

Dans un autoclave en acier inoxydable préalablement purgé à l'argon, on introduit 39,1 mg de $[RhCl(cyclooctadiène\text{-}1,5)]_2$ (0,16 milliatome-gramme de rhodium), 0,33 g de TPPTS Na (0,49 milliatome-gramme de $P^{3+}$), 0,15 g de $Na_2CO_3$ (1,4 millimole) et 10 $cm^3$ d'$H_2O$.

On introduit ensuite 24,6 millimoles de myrcène (3,34 g) et 38,8 millimoles de pentanedione-2,4 (3,9 g).

On chauffe à 80 °C sous agitation pendant 24 heures.

On obtient 3,19 g de $a_3 + a'_3$ (mélange équimolaire) soit un taux de transformation du myrcène de 55,5 %, de la pentanedione-2,4 de 35,2 %, la sélectivité en $a_3 + a'_3$ étant de 98,5 %.

Essai comparatif

En opérant comme ci-dessus mais en utilisant 24 mg de $PdCl_2$ (0,14 milliatome-gramme de palladium) au lieu du $[RhCl(cyclooctadiène\text{-}1,5)]_2$. On obtient après élimination des réactifs n'ayant pas réagi, 1 g de produit organique constitué essentiellement de dimères du myrcène. On n'observe pas la présence des composés $a_3$ et $a'_3$.

Exemple 16

On opère comme dans l'exemple 15 mais en opérant à 120 °C pendant 3 heures.

On obtient 3,8 g de $a_3 + a'_3$ (mélange équimolaire).

Le taux de transformation du myrcène est de 66,9 %, celui de la pentanedione-2,4 est de 41,7 %, la sélectivité en $a_3 + a'_3$ étant de 98,5 %.

Exemple 17

On opère comme dans l'exemple 15 mais en utilisant 1,06 g de TPPTS Na (1,57 milliatome-gramme de $p^{3+}$) à 120 °C pendant 3 heures.

On obtient 3,7 g de $a_3 + a'_3$ (mélange équimolaire).

Le taux de transformation du myrcène est de 63,1 %, celui de la pentanedione-2,4 est de 39,7 %, la sélectivité en $a_3 + a'_3$ étant de 99 %.

Exemple 18 à 22

Addition sur le myrcène de l'acétylacétate d'éthyle pour obtenir :

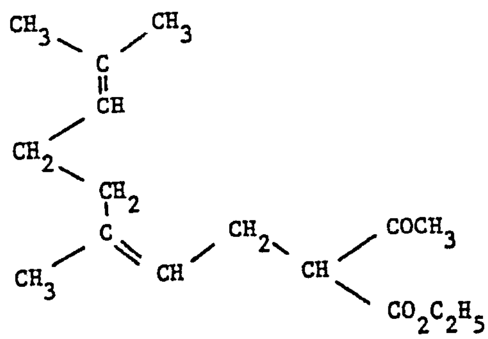

($a_4$)

et

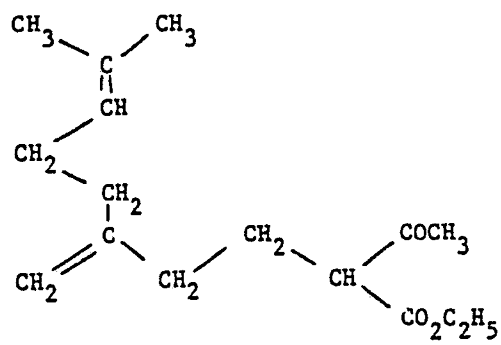

($a'_4$)

Exemple 18

Dans un autoclave en acier inoxydable préalablement purgé à l'argon, on introduit 100 mg de $[RhCl(cyclooctadiène\text{-}1,5)]_2$ (0,41 milliatome-gramme de rhodium) 0,78 g de TPPTS Na (1,15 milliatome-gramme de $P^{3+}$) 0,156 g de $Na_2CO_3$ (1,5 millimole) et 30 $cm^3$ d'$H_2O$.

On introduit ensuite 64,7 millimoles de myrcène (8,8 g) et 127 millimoles d'acétylacétate d'éthyle (16,5 g).

On chauffe sous agitation à 100 °C pendant 6 heures.

On obtient 12,66 g de $a_4 + a'_4$ (mélange équimolaire).

Le taux de transformation du myrcène est de 80 %, celui de l'acétylacétate d'éthyle est de 40 %, la sélectivité en $a_4 + a'_4$ étant de 99 %.

Exemple 19

On opère comme dans l'exemple 18 mais en utilisant 49 mg de $[RhCl(cyclooctadiène\text{-}1,5)]_2$ (0,2 milliatome-gramme de rhodium), 1,42 g de TPPTS Na (2,1 milliatomes-gramme de $P^{3+}$), 70 mg de $Na_2CO_3$ (0,65 millimole) et 30 $cm^3$ d'$H_2O$, 82 millimoles de myrcène (11,15 g) et 117 millimoles d'acétylacétate d'éthyle (15,2 g).

On chauffe à 150 °C sous agitation pendant 1 heure.

On obtient 6,07 g de $a_4 + a'_4$ (mélange équimolaire).

Le taux de transformation du myrcène est de 33 %, celui de l'acétylacétate d'éthyle est de 23,2 %, la sélectivité étant de 99 %.

Exemple 20

Addition sur le myrcène de la phénylsulfonylacétone pour obtenir :

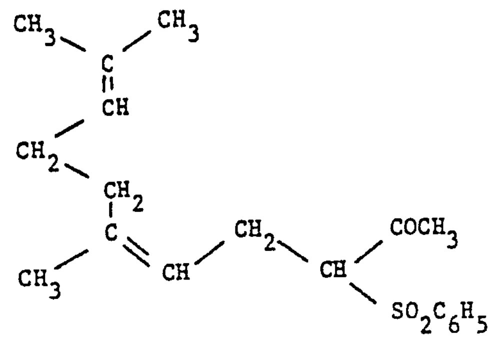

($a_5$)

et

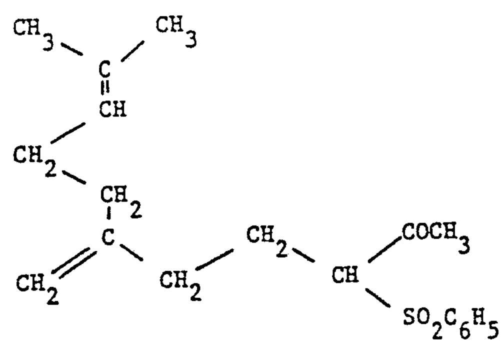

($a'_5$)

Dans un autoclave en acier inoxydable préalablement purgé à l'argon, on introduit 67,6 mg de $[RhCl(cyclooctadiène\text{-}1,5)]_2$ (0,27 milliatome-gramme de rhodium), 0,64 g de TPPTS Na (0,95 milliatome-gramme de $P^{3+}$) 0,30 g de $Na_2CO_3$ (2,8 millimoles) et 20 $cm^3$ d'$H_2O$.

On introduit ensuite 81,2 millimoles de myrcène (11,05 g) et 80 millimoles de phénylsulfonylacétone (15,84 g).

On chauffe à 100 °C pendant 6 heures sous agitation.

On obtient :

— 8,2 g de $a_5$
— 10,01 g de $a'_5$

soit un taux de transformation du myrcène de 67,1 %, de la phénylsulfonylacétone de 68,1 %, la sélectivité en $a_5 + a'_5$ étant de 99 %.

Exemple 21

Addition sur le β-farnésène de l'acétylacétate d'éthyle pour obtenir :

(Voir Schéma, page 13)

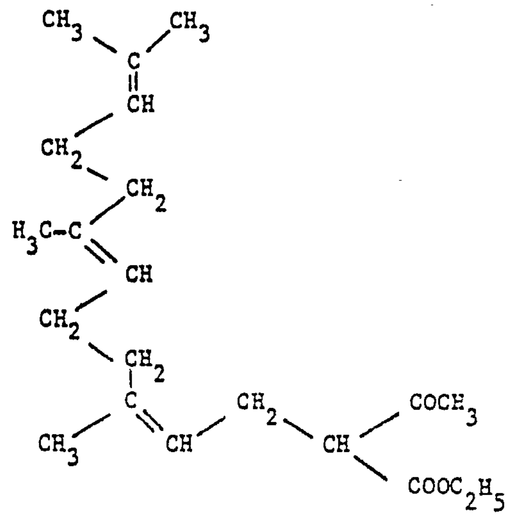

($a_6$)

et

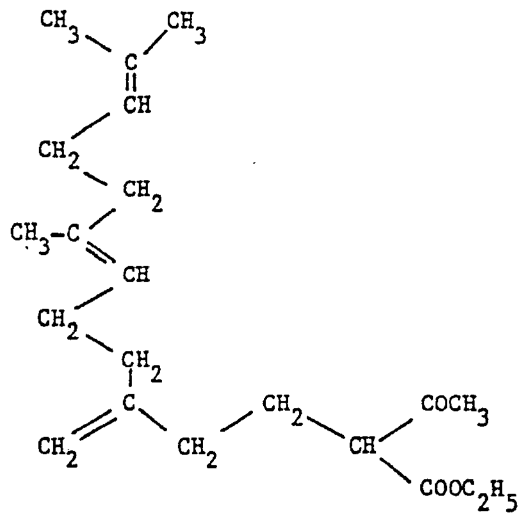

($a'_6$)

Dans un autoclave en acier inoxydable préalablement purgé à l'argon, on introduit 64 mg de [RhCl(cyclooctadiène-1,5)]$_2$ (0,26 milliatome-gramme de rhodium), 0,53 g de TPPTS Na (0,78 milliatome-gramme de $P^{3+}$), 0,20 g de $Na_2CO_3$ (1,9 millimoles) et 15 cm$^3$ d'$H_2O$.

On introduit ensuite 16 millimoles de β-farnésène (3,26 g) et 58,9 millimoles d'acétylacétate d'éthyle (7,66 g).

On chauffe à 120 °C pendant 6 heures sous agitation.

On obtient 2,34 g de $a_6$ + $a'_6$ (mélange équimolaire).

Le taux de transformation du β-farnésène est de 43,7 %, celui de l'acétylacétate d'éthyle est de 11,9 %, la sélectivité en $a_6'$ + $a'_6$ étant de 99 %.

Exemple 22

Addition de l'isoprène sur l'acétylacétate d'éthyle pour obtenir $a_1$ et $a'_1$.

Dans un autoclave en acier inoxydable préalablement purgé à l'argon, on introduit 41,6 mg de [RhCl(cyclooctadiène-1,5)]$_2$ (0,17 milliatome-gramme de rhodium), 0,58 g de TPPTS Na (0,86 milliatome-gramme de $P^{3+}$), 0,44 g de triéthylamine $N(C_2H_5)_3$ (4,35 millimoles) et 30 cm$^3$ d'$H_2O$.

On introduit ensuite 99,8 millimoles d'isoprène (6,78 g) et 126 millimoles d'acétylacétate d'éthyle (16,38 g).

On chauffe sous agitation à 100 °C pendant 3 heures.

On obtient :

— 6,05 g de $a_1$
— 12,50 g de $a'_1$

soit un taux de transformation de l'isoprène de 94,8 %, de l'acétylacétate d'éthyle de 75,1 %, la sélectivité en $a_1$ + $a'_1$ étant de 99 %.

Exemple 23

Addition de l'isoprène sur l'éthoxycarbonyl-2 cyclohexanone pour obtenir :

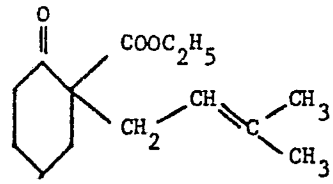

(a$_7$)

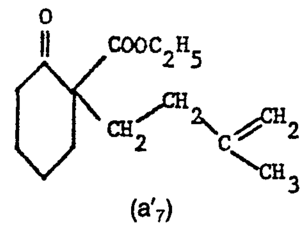

(a'$_7$)

Dans un autoclave en acier inoxydable préalablement purgé à l'argon, on introduit 66 mg de [RhCl(cyclooctadiène-1,5)]$_2$ soit 0,26 milliatome-gramme de rhodium, 0,85 g de TPPTSNa (1,33 milliatome-gramme de $P^{3+}$), 0,24 g de $Na_2CO_3$ (2,26 millimoles) et 15 cm$^3$ d'eau. On introduit ensuite 81,5 millimoles d'isoprène (5,55 g) et 92,9 millimoles d'éthoxycarbonyl-2 cyclohexanone (15,81 g).

On chauffe à 100 °C sous agitation pendant 3 heures.

On obtient :

— 4,9 g de a$_7$
— 4,95 g de a'$_7$,

soit un taux de transformation de l'isoprène de 50,7 % de l'éthoxycarbonyl-2 cyclohexanone de 44,5 %, la sélectivité en a$_7$ + a'$_7$ étant de 99 %.

Exemple 24

Addition sur l'isoprène de l'hydroxyacétone pour obtenir :

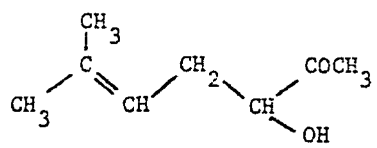

(a$_8$)

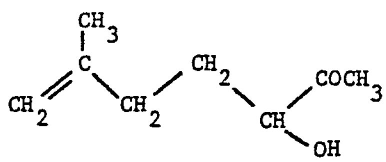

(a'$_8$)

Dans un autoclave en acier inoxydable préalablement purgé à l'argon, on introduit 66 mg de [RhCl(cyclooctadiène-1,5)]$_2$ soit 0,26 milliatome-gramme de rhodium, 0,90 g de TPPTSNa (1,34 milliatome-gramme de $P^{+3}$), 0,25 g de $Na_2CO_3$ (2,38 millimoles) et 15 cm$^3$ d'eau. On introduit ensuite 104,7 millimoles d'isoprène (7,13 g) et 128 millimoles d'hydroxyacétone (9,49 g). On chauffe à 100 °C sous agitation pendant 16 heures.

On obtient 1,77 g d'un produit contenant 70 % d'un mélange équimoléculaire de a$_8$ + a'$_8$ (soit un taux de transformation de l'isoprène de 10 %).

Exemple 25

Addition sur l'isoprène de l'acétoïne pour obtenir :

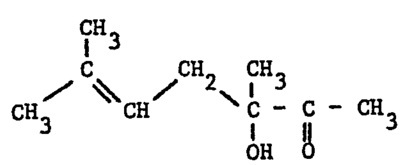

(a$_9$)

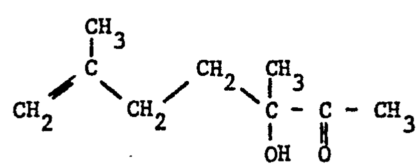

(a'$_9$)

Dans un autoclave en acier inoxydable préalablement purgé à l'argon, on introduit 64,8 mg de $[RhCl(cyclooctadiène\text{-}1,5)]_2$ soit 0,26 milliatome-gramme de rhodium, 0,90 g de TPPTSNa (1,34 milliatome-gramme de $P^{+3}$), 0,25 g de $Na_2CO_3$ (2,36 millimoles), 11 g d'acétoïne (124,8 millimoles) et 15 $cm^3$ d'eau. On introduit ensuite 103,8 millimoles d'isoprène (7,07 g) et on chauffe à 100 °C sous agitation pendant 16 heures.

On obtient 3,18 g de $a_9 + a'_9$, soit un taux de conversion de l'isoprène de 19,6 %.

Exemple 26

Influence d'un alcool — Addition sur le myrcène de l'acétylacétate de méthyle pour obtenir :

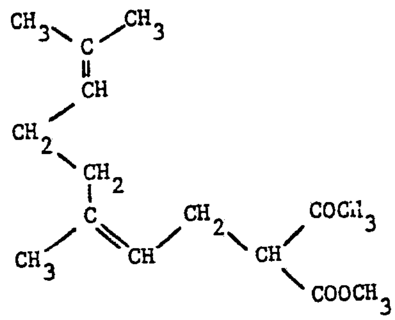

($a_{10}$)

et

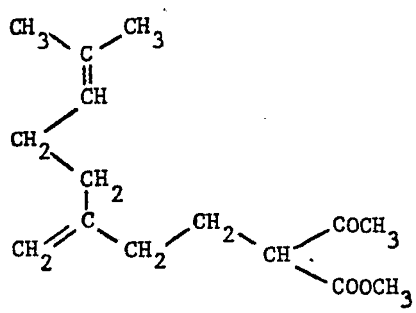

($a'_{10}$)

a) sans méthanol

Dans un réacteur en acier inoxydable préalablement purgé à l'argon, on introduit 41,1 mg de $[RhCl(cyclooctadiène\text{-}1,5)]_2$ (0,167 milliatome-gramme de rhodium), 2,2 g de TPPTSNa (3,256 milliatome-gramme de $P^{+3}$), 72 mg de $Na_2CO_3$ (0,68 millimole) et 20 $cm^3$ d'eau.

On introduit ensuite 117 millimoles de myrcène (15,92 g) et 120 millimoles d'acétylacétate de méthyle (13,9 g).

On chauffe sous agitation à 90 °C pendant 1 heure.

On obtient 7,376 g d'un mélange équimoléculaire de $b_4 + b'_4$. Le taux de transformation du myrcène est de 24,9 %, celui de l'acétylacétate de méthyle de 24,4 %, la sélectivité en $a_{10} + a'_{10}$ étant de 99 %.

b) avec méthanol

Dans un réacteur en acier inoxydable préalablement purgé à l'argon, on introduit 40,8 mg de $[RhCl(cyclooctadiène\text{-}1,5)]_2$ (0,165 milliatome-gramme de rhodium), 2,2 g de TPPTSNa (3,256 milliatome-gramme de $P^{+3}$), 78,2 mg de $Na_2CO_3$ (0,68 millimole), 15 $cm^3$ d'eau et 5 $cm^3$ de méthanol.

On introduit ensuite 117 millimoles de myrcène (15,92 g) et 120 millimoles d'acétylacétate de méthyle (13,9 g).

On chauffe sous agitation à 90 °C pendant 1 heure.

On obtient 12,488 g d'un mélange équimoléculaire de $a_{10} + a'_{10}$. Le taux de transformation du myrcène est de 42,4 %, celui de l'acétylacétate de méthyle de 41,3 %, la sélectivité en $a_{10} + a'_{10}$ étant de 99 %.

**Revendications**

1. Procédé d'addition d'un composé ayant un carbone activé sur un butadiène-1,3 portant un substituant hydrocarboné sur l'atome de carbone 2, procédé du type selon lequel on fait réagir un composé ayant un atome de carbone activé de formule générale :

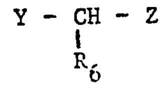

dans laquelle $R_6$ représente un atome d'hydrogène ou un radical hydrocarboné et l'un des symboles Y ou Z représente un groupement électro-attracteur et l'autre représente un groupement électro-attracteur ou électro-donneur, et, lorsque l'un des symboles Y ou Z représente un radical acyle, celui-ci peut former avec $R_6$ un cycle saturé à 5 ou 6 chaînons, et le butadiène portant un substituant hydrocarboné de formule générale :

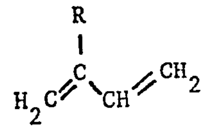

dans laquelle R est choisi parmi le groupe comprenant les radicaux alkyle ayant de 1 à 20 atomes de carbone et les radicaux phényle et naphtyle éventuellement substitués par des radicaux alkyle ayant de 1 à 20 atomes de carbone, dans l'eau ou dans un mélange hydro-alcoolique contenant au maximum 50 % d'un alcool aliphatique contenant 1 à 3 atomes de carbone en présence d'un catalyseur constitué, d'une part, par au moins une phosphine soluble dans l'eau et, d'autre part, par au moins un composé de métal de transition, le catalyseur étant en solution dans l'eau ou dans le mélange hydro-alcoolique, procédé caractérisé en ce que, dans le but d'obtenir une addition totalement sélective du composé ayant un atome de carbone activé sur le carbone 4 du butadiène-1,3 portant un substituant hydrocarboné sur le carbone 2, le métal de transition mis en œuvre est le rhodium.

2. Procédé selon la revendication 1 caractérisé en ce que le rhodium est mis en œuvre sous forme d'un composé choisi parmi le groupe comprenant les sels inorganiques, organiques et les complexes du rhodium.

3. Procédé selon la revendication 2 caractérisé en ce que le composé du rhodium est choisi parmi le groupe comprenant $RhCl_3$, $RhBr_3$, $Rh_2O$, $Rh_2O_3$, $Rh(NO_3)_3$, $Rh(CH_3COO)_3$, $Rh(CH_3COCHCOCH_3)_3$, $[RhCl(\text{cyclooctadiène-1,5})]_2$, $[RhCl(CO_2)]_2$, $RhCl_3(C_2H_5NH_2)_3$.

4. Procédé selon la revendication 2 caractérisé en ce que le composé du rhodium est $RhCl_3$ ou $[RhCl(\text{cyclooctadiène-1,5})]_2$.

5. Procédé selon l'une quelconque des revendications précédentes caractérisé en ce que l'on met en œuvre une quantité de composé du rhodium telle que le nombre d'atomes-gramme de rhodium élémentaire par litre de solution réactionnelle est compris entre $10^{-4}$ et 1.

6. Procédé selon la revendication 5 caractérisé en ce que le nombre d'atomes-gramme de rhodium élémentaire par litre de solution réactionnelle est compris entre 0,001 et 0,5.

7. Procédé selon l'une quelconque des revendications précédentes caractérisé en ce que la quantité de phosphine est telle que le nombre d'atomes-gramme de phosphore trivalent rapporté à un atome-gramme de rhodium soit compris entre 0,1 et 200.

8. Procédé selon la revendication 7 caractérisé en ce que le nombre d'atomes-gramme de phosphore trivalent rapporté à un atome-gramme de rhodium est compris entre 3 et 100.

9. Procédé selon la revendication 1 caractérisé en ce que dans le butadiène-1,3 portant un substituant hydrocarboné sur le carbone 2, les radicaux alkyle ayant de 1 à 20 atomes de carbone sont choisis parmi les radicaux $C_nH_{2n+1}$, $C_nH_{2n-1}$, $C_nH_{2n-3}$ et $C_nH_{n-1}$ où n est compris entre 1 et 20.

10. Procédé selon la revendication 9 caractérisé en ce que le dérivé du butadiène est l'isoprène, le myrcène ou le β-farnésène.

11. Procédé selon la revendication 1 caractérisé en ce que $R_6$ représente un radical alcoyle contenant 1 à 20 atomes de carbone ou un radical phényle ou naphtyle éventuellement substitué par un ou plusieurs radicaux alcoyles contenant 1 à 20 atomes de carbone.

12. Procédé selon la revendication 1 caractérisé en ce que dans le composé ayant un atome de carbone activé les groupements électroattracteurs sont choisis parmi le groupe comprenant les radicaux de formules CHO, $COR_1$, $CO_2R_2$, $SO_2R_3$, $CON(R_4R_5)$, CN, $NO_2$ et le groupement électrodonneur est choisi parmi le groupe comprenant les radicaux de formule $NHCOR_7$, $OR_8$, OH, $OCOR_9$, $SR_{10}$, SH et les atomes d'halogène, dans lesquels $R_1$, $R_2$, $R_3$, $R_4$, $R_5$, $R_7$, $R_8$, $R_9$ et $R_{10}$ représentent chacun un groupement hydrocarboné contenant 1 à 12 atomes de carbone.

13. Procédé selon la revendication 12 caractérisé en ce que le composé ayant un atome de carbone activé est choisi parmi la pentanedione-2,4, l'acétylacétate de méthyle, l'acétylacétate d'éthyle, la phénylsulfonylacétone, l'hydroxyacétone, l'acétoïne ou l'éthoxycarbonyl-2 cyclohexanone.

14. Procédé selon l'une des revendications précédentes caractérisé en ce que l'on ajoute au mélange réactionnel un réducteur du rhodium.

15. Procédé selon l'une des revendications précédentes caractérisé en ce que l'on ajoute au mélange réactionnel une base.

16. Procédé selon l'une des revendications précédentes caractérisé en ce que la température est inférieure à 200 °C.

**Claims**

1. Process for adding a compound having an activated carbon onto a buta-1,3-diene carrying a hydrocarbon substituent on the 2 carbon atom, which process is of the type wherein a compound having an activated carbon atom, of the general formula :

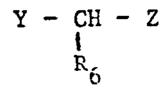

in which $R_6$ represents a hydrogen atom or a hydrocarbon radical and one of the symbols Y or Z represents an electron-attracting group and the other represents an electron-attracting or an electron-donating group, and, if one of the symbols Y or Z represents an acyl radical, this can form a 5-membered or 6-membered saturated ring with $R_6$, is reacted with butadiene carrying a hydrocarbon substituent, of the general formula :

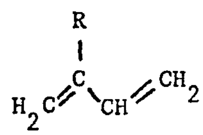

in which R is chosen from amongst the group comprising alkyl radicals having from 1 to 20 carbon atoms and phenyl and naphthyl radicals optionally substituted by alkyl radicals having from 1 to 20 carbon atoms, in water or in an aqueous-alcoholic mixture containing at most 50 % of an aliphatic alcohol containing 1 to 3 carbon atoms, in the presence of a catalyst consisting on the one hand of at least one water-soluble phosphine and on the other hand of at least one transition metal compound, the catalyst being dissolved in water or in the aqueous-alcoholic mixture, the said process being characterised in that, in order to achieve a totally selective addition of the compound having an activated carbon atom onto the 4 carbon of the buta-1,3-diene carrying a hydrocarbon substituent on the 2 carbon, the transition metal used is rhodium.

2. Process according to Claim 1, characterised in that the rhodium is used in the form of a compound chosen from amongst the group comprising the inorganic salts, the organic salts and the complexes of rhodium.

3. Process according to Claim 2, characterised in that the rhodium compound is chosen from amongst the group comprising $RhCl_3$, $RhBr_3$, $Rh_2O$, $Rh_2O_3$, $Rh(NO_3)_3$, $Rh(CH_3COO)_3$, $Rh(CH_3COCH\text{-}COCH_3)_3$, $[RhCl(\text{cycloocta-1,5-diene})]_2$, $[RhCl(CO_2)]_2$ and $RhCl_3(C_2H_5NH_2)_3$.

4. Process according to Claim 2, characterised in that the rhodium compound is $RhCl_3$ or $[RhCl(\text{cycloocta-1,5-diene})]_2$.

5. Process according to any one of the preceding claims, characterised in that the amount of rhodium compound used is such that the number of gram atoms of elementary rhodium per litre of reaction solution is between $10^{-4}$ and 1.

6. Process according to Claim 5, characterised in that the number of gram atoms of elementary rhodium per litre of reaction solution is between 0.001 and 0.5.

7. Process according to any one of the preceding claims, characterised in that the amount of phosphine is such that the number of gram atoms of trivalent phosphorus, relative to one gram atom of rhodium, is between 0.1 and 200.

8. Process according to Claim 7, characterised in that the number of gram atoms of trivalent phosphorus, relative to one gram atom of rhodium, is between 3 and 100.

9. Process according to Claim 1, characterised in that, in the buta-1,3-diene carrying a hydrocarbon substituent on the 2 carbon, the alkyl radicals having from 1 to 20 carbon atoms are chosen from amongst the radicals $C_nH_{2n+1}$, $C_nH_{2n-1}$, $C_nH_{2n-3}$ and $C_nH_{n-1}$, in which n is between 1 and 20.

10. Process according to Claim 9, characterised in that the butadiene derivative is isoprene, myrcene or β-farnesene.

11. Process according to Claim 1, characterised in that $R_6$ represents an alkyl radical containing 1 to 20 carbon atoms or a phenyl or naphthyl radical optionally substituted by one or more alkyl radicals containing 1 to 20 carbon atoms.

12. Process according to Claim 1, characterised in that, in the compound having an activated carbon atom, the electron-attracting groups are chosen from amongst the group comprising the radicals of the formulae CHO, $COR_1$, $CO_2R_2$, $SO_2R_3$, $CON(R_4R_5)$, CN and $NO_2$ and the electron-donating group in chosen from amongst the group comprising the radicals of the formulae $NHCOR_7$, $OR_8$, OH, $OCOR_9$, $SR_{10}$, SH and halogen atoms, in which radicals $R_1$, $R_2$, $R_3$, $R_4$, $R_5$, $R_7$, $R_8$, $R_9$ and $R_{10}$ each represent a hydrocarbon group containing 1 to 12 carbon atoms.

13. Process according to Claim 12, characterised in that the compound having an activated carbon atom is chosen from amongst pentane-2,4-dione, methyl acetylacetate, ethyl acetylacetate, phenylsulfonylacetone, hydroxyacetone, acetoin or 2-ethoxycarbonylcyclohexanone.

14. Process according to one of the preceding claims, characterised in that a reducing agent for the rhodium is added to the reaction mixture.

15. Process according to one of the preceding claims, characterised in that a base is added to the reaction mixture.

16. Process according to one of the preceding claims, characterised in that the temperature is below 200 °C.

**Ansprüche**

1. Verfahren zur Anlagerung einer Verbindung mit einem aktivierten Kohlenstoffatom an einen Kohlenwasserstoffsubstituenten am in 2-Stellung befindlichen Kohlenstoffatom tragenden 1,3-Butadien, wobei man eine Verbindung mit einem aktivierten Kohlenstoffatom der allgemeinen Formel :

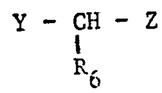

worin $R_6$ ein Wasserstoffatom oder ein Kohlenwasserstoffrest ist und das eine der Symbole Y oder Z eine Elektronenakzeptorgruppe und das andere eine Elektronenakzeptorgruppe oder eine Elektronendonatorgruppe ist und, wenn das eine der Symbole Y oder Z ein Acylrest ist, dieser selbst mit $R_6$ einen mit 5 oder 6 Ketten gesättigten Ring bilden kann, mit dem Butadien, das einen Kohlenwasserstoffsubstituenten der allgemeinen Formel :

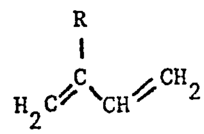

trägt, worin R ausgewählt ist aus der Gruppe, die Alkylreste mit 1-20 Kohlenstoffatomen und gegebenenfalls durch Alkylreste mit 1-20 Kohlenstoffatomen substituierte Phenyl- und Naphthylreste umfasst, in Wasser oder in einem wässerig-alkoholischen Gemisch, das höchstens 50 % eines aliphatischen Alkohols mit 1-3 Kohlenstoffatomen enthält, in Gegenwart eines Katalysators umsetzt, der einerseits aus mindestens einem in Wasser löslichen Phosphin und anderseits aus mindestens einer Übergangsmetallverbindung besteht, und in Wasser oder in dem wässerig-alkoholischen Gemisch in Lösung ist, dadurch gekennzeichnet, daß zur vollständigen selektiven Anlagerung der Verbindung mit einem aktivierten Kohlenstoffatom an das in 4-Stellung befindliche Kohlenstoffatom des einen Kohlenwasserstoffsubstituenten am in 2-Stellung befindlichen Kohlenstoffatom tragenden 1,3-Butadiens als Übergangsmetall Rhodium eingesetzt wird.

2. Verfahren nach Anspruch 1, dadurch gekennzeichnet, daß das Rhodium in Form einer Verbindung eingesetzt wird, die aus der Gruppe ausgewählt ist, die anorganische Salze, organische Salze und die Komplexe von Rhodium umfasst.

3. Verfahren nach Anspruch 2, dadurch gekennzeichnet, daß die Rhodiumverbindung aus der Gruppe aus $RhCl_3$, $RhBr_3$, $Rh_2O$, $Rh_2O_3$, $Rh(NO_3)_3$, $Rh(CH_3COO)_3$, $Rh(CH_3COCHCOCH_3)_3$, $[RhCl(1,5\text{-Cyclooctadien})]_2$, $[RhCl(CO_2)]_2$, $RhCl_3(C_2H_5NH_2)_3$ ausgewählt wird.

4. Verfahren nach Anspruch 2, dadurch gekennzeichnet, daß die Rhodiumverbindung $RhCl_3$ oder $[RhCl(1,5\text{-Cyclooctadien})]_2$ ist.

5. Verfahren nach einem der vorhergehenden Ansprüche, dadurch gekennzeichnet, daß man eine solche Menge an Rhodiumverbindung einsetzt, daß die Anzahl von Grammatomen elementaren Rhodiums pro Liter Reaktionslösung zwischen $10^{-4}$ und 1 beträgt.

6. Verfahren nach Anspruch 5, dadurch gekennzeichnet, daß die Anzahl von Grammatomen elementaren Rhodiums pro Liter Reaktionslösung zwischen 0,001 und 0,5 beträgt.

7. Verfahren nach einem der vorhergehenden Ansprüche, dadurch gekennzeichnet, daß die Phosphinmenge derart ist, daß die Anzahl Grammatome von dreiwertigem Phosphor, bezogen auf ein Grammatom Rhodium, zwischen 0,1 und 200 beträgt.

8. Verfahren nach Anspruch 7, dadurch gekennzeichnet, daß die Anzahl Grammatome von dreiwertigem Phosphor, bezogen auf ein Grammatom Rhodium, zwischen 3 und 100 beträgt.

9. Verfahren nach Anspruch 1, dadurch gekennzeichnet, daß bei einem Kohlenwasserstoffsubstituenten am in 2-Stellung befindlichen Kohlenstoffatom tragenden 1,3-Butadien die Alkylreste mit 1-20 Kohlenstoffatomen aus den Resten $C_nH_{2n+1}$, $C_nH_{2n-1}$, $C_nH_{2n-3}$ und $C_nH_{n-1}$ ausgewählt werden, worin n zwischen 1 und 20 ist.

10. Verfahren nach Anspruch 9, dadurch gekennzeichnet, daß das Butadienderivat Isopren, Myrcen oder β-Farnesen ist.

11. Verfahren nach Anspruch 1, dadurch gekennzeichnet, daß $R_6$ einen Alkylrest mit 1-20 Kohlenstoffatomen oder einen gegebenenfalls durch ein oder mehrere Alkylreste mit 1-20 Kohlenstoffatomen substituierten Phenyl- oder Naphthylrest bedeutet.

12. Verfahren nach Anspruch 1, dadurch gekennzeichnet, daß bei der Verbindung mit einem aktivierten Kohlenstoffatom die Elektronenakzeptorgruppen aus der Gruppe aus den Resten der Formel CHO, $COR_1$, $CO_2R_2$, $SO_2R_3$, $CON(R_4R_5)$, CN, $NO_2$ ausgewählt werden und die Elektronendonatorgruppe aus der Gruppe aus den Resten der Formel $NHCOR_7$, $OR_8$, OH, $OCOR_9$, $SR_{10}$, SH und den Halogenatomen ausgewählt wird, worin $R_1$, $R_2$, $R_3$, $R_4$, $R_5$, $R_7$, $R_8$, $R_9$ und $R_{10}$ jeweils eine Kohlenwasserstoffgruppe mit 1-12 Kohlenstoffatomen bedeuten.

13. Verfahren nach Anspruch 12, dadurch gekennzeichnet, daß die Verbindung mit einem aktivierten Kohlenstoffatom ausgewählt wird aus 2,4-Pentandion, Methylacetylacetat, Äthylacetylacetat, Phenylsulfonylaceton, Hydroxyaceton, Acetoin oder 2-Äthoxycarbonylcyclohexanon.

14. Verfahren nach einem der vorhergehenden Ansprüche, dadurch gekennzeichnet, daß man dem Reaktionsgemisch ein Rhodiumreduktionsmittel zusetzt.

15. Verfahren nach einem der vorhergehenden Ansprüche, dadurch gekennzeichnet, daß man dem Reaktionsgemisch eine Base zusetzt.

16. Verfahren nach einem der vorhergehenden Ansprüche, dadurch gekennzeichnet, daß die Temperatur niedriger als 200 °C ist.